# EUROPÄISCHE PATENTANMELDUNG

(11) **EP 4 371 648 A1**
(43) Veröffentlichungstag der Anmeldung: **22.05.2024**
(21) Anmeldenummer: 22211367.2
(22) Anmeldetag: 05.12.2022
(51) Int. Cl.: B01D 53/047, A61M 16/10, B01D 53/053

(54) **SAUERSTOFFBEREITSTELLUNGSSYSTEM UND VERFAHREN**

(30) Priorität: 16.11.2022 DE 102022130305
(71) Anmelder: Özkalp, Harun, 22085 Hamburg (DE)
(72) Erfinder: Özkalp, Harun, 22085 Hamburg (DE)
(74) Vertreter: Marschall, Stefan

(57) **Zusammenfassung**

Offenbart ist ein Sauerstoffbereitstellungssystem mit einem mobilen Sauerstoffgaserzeugungsgerät 1. Das Sauerstoffgaserzeugungsgerät 1 umfasst mindestens eine Adsorbereinrichtung 10, die dazu eingerichtet ist, mittels Druckwechsel-Adsorption aus Umgebungsluft L ein Gas S zu erzeugen, das zu mindestens 90 Vol.-% aus Sauerstoff besteht. Darüber hinaus umfasst das Sauerstoffgaserzeugungsgerät 1 mindestens einen Verdichter 13 zum Verdichten des erzeugten Gases S, mindestens einen Speicher 14 zur Aufnahme des vom mindestens einen Verdichter verdichteten Gases und einen oder mehrere Auslässe 15 zur Abgabe des erzeugten Gases aus dem mindestens einen Speicher 14.

Offenbart ist ferner ein Verfahren Bereitstellen eines Sauerstoffgases S, wobei das Verfahren ein Betreiben eines Sauerstoffbereitstellungssystems umfasst.

## Beschreibung

Die vorliegende Erfindung betrifft ein Sauerstoffbereitstellungssystem mit einem mobilen Sauerstoffgaserzeugungsgerät, mit dem ein Sauerstoffgas, also ein Gas mit einem im Vergleich zur Umgebungsluft erhöhten Sauerstoffgehalt bereitgestellt werden kann, insbesondere zur medizinischen Verwendung. Darüber hinaus betrifft die Erfindung ein Verfahren zur Bereitstellung von Sauerstoffgas.

Zur mobilen Bereitstellung von Sauerstoffgas können herkömmlicherweise Sauerstoffflaschen verwendet werden. Im Bereich der medizinischen Sauerstoffbehandlung sind zudem tragbare Geräte zur individuellen Versorgung von Patienten bekannt, die das Sauerstoffgas mittels Druckwechsel-Adsorption erzeugen. Derartige Geräte sind insbesondere dazu ausgelegt, die Bewegungsfreiheit der einzelnen Patienten zu erhöhen bzw. zu verbessern.

In der DE 36 27 203 C1 ist eine Vorrichtung offenbart, die ein Sauerstoffanreicherungs- und - verteilungssystem mit einem Druckwechseladsorber zur Versorgung einer Gruppe von Menschen in einem Schutzraum umfasst.

Der vorliegenden Erfindung liegt die Aufgabe zugrunde, eine Technik bereitzustellen, mit der die mobile Bereitstellung von hoch sauerstoffhaltigem Gas auf besonders zuverlässige Weise auch unter schwierigen Bedingungen erleichtert wird, wie sie beispielsweise aufgrund von nicht vorhandener Infrastruktur in entlegenen Gebieten (z.B. in Afrika oder Australien (Outback)) oder bei zerstörter oder stark beschädigter Infrastruktur nach Naturkatastrophen oder sonstigen Unglücken vorliegen können.

Die Aufgabe wird gelöst durch ein Sauerstoffbereitstellungssystem gemäß Anspruch 1 und durch ein Verfahren gemäß Anspruch 15. Vorteilhafte Ausführungsformen sind in den Unteransprüchen, der Beschreibung und in den Figuren offenbart.

Ein erfindungsgemäßes Sauerstoffbereitstellungssystem umfasst ein mobiles Sauerstoffgaserzeugungsgerät, das also für einen wiederholten Transport und eine Verwendung an wechselnden Orten konzipiert ist. Vorzugsweise ist das Sauerstoffbereitstellungssystem insgesamt in diesem Sinne mobil.

Das Sauerstoffgaserzeugungsgerät umfasst mindestens eine Adsorbereinrichtung, also eine einzelne Adsorbereinrichtung oder mehrere Adsorbereinrichtungen. Die mindestens eine Adsorbereinrichtung ist dazu eingerichtet, mittels Druckwechsel-Adsorption aus (der Adsorbereinrichtung zugeführter) Umgebungsluft ein Gas zu erzeugen, das zu mindestens 90 Vol.-% aus Sauerstoff besteht; insbesondere ist die Adsorbereinrichtung also ein Sauerstoffkonzentrator. Im Falle mehrerer Adsorbereinrichtungen können diese einzeln (nacheinander) und/oder synchron (also parallel) zu betreiben sein, vorzugsweise nach Wahl eines Verwenders.

Das Sauerstoffbereitstellungssystem weist weiterhin mindestens einen Verdichter zum Verdichten des erzeugten (Sauerstoff-)Gases sowie mindestens einen Speicher auf, der dazu eingerichtet ist, das verdichtete erzeugte Gas aufzunehmen. Über mindestens einem Auslass des Sauerstoffgaserzeugungsgeräts kann das erzeugte Gas aus dem mindestens einen Speicher abgegeben werden. Vorzugsweise ist das Sauerstoffgaserzeugungsgerät dabei dazu eingerichtet, das erzeugte Gas am Auslass bzw. an mindestens einem der mehreren Auslässe abzugeben, während gleichzeitig (von der mindestens einen Adsorbereinrichtung) eine Durchführung der Druckwechsel-Adsorption und damit eine Gaserzeugung fortgesetzt wird.

Das erfindungsgemäße Sauerstoffbereitstellungssystem stellt somit eine Einheit dar, mit der aus Umgebungsluft Sauerstoffgas erzeugt und bereitgestellt werden kann, insbesondere zur medizinischen, aber auch zur industriellen Verwendung. Es ist vorzugsweise elektrisch betrieben. Mit einer entsprechenden Stromversorgung (wie einem Stromgenerator oder einer Batterie), die zum Sauerstoffbereitstellungssystem gehören kann, bildet es eine autarke Vorrichtung zur Sauerstoffversorgung, die damit insbesondere in Notfällen unter den oben genannten Extrembedingungen vorteilhaft eingesetzt werden kann.

Insbesondere wird mit dem erfindungsgemäßen Sauerstoffbereitstellungssystem eine mobil einsetzbare Technologie bereitgestellt, die zuverlässig eine medizinisch hochwertige Notfallversorgung von Patienten mit Sauerstoff auch in unwegsamem Gebiet und in Fällen ermöglicht, in denen die jeweilige Infrastruktur einen Transport von Flüssigsauerstoff in O₂-Zylindern, wie er herkömmlicherweise für Notfälle vorgesehen ist, erschwert oder sogar unmöglich macht.

Der mindestens eine Speicher ermöglicht dabei ein Abpuffern eventueller Stromschwankungen oder sogar Stromausfälle. Die vorgeschaltete Verdichtung des erzeugten Gases erlaubt zum einen eine volumensparende Ausführung des mindestens einen Speichers, was den Transport des Sauerstoffbereitstellungssystems erleichtert, zum anderen kann durch sie eine Abgabe des erzeugten Gases aus dem Auslass bzw. aus mindestens einem der Auslässe bei gegenüber der Umgebung erhöhtem Druck sichergestellt werden.

Gemäß vorteilhaften Ausführungsformen ist der mindestens eine Verdichter dazu eingerichtet, das erzeugte Gas auf einen Druck von mindestens 5 bar, mindestens 5,5 bar, mindestens 6 bar oder sogar mindestens 6,5 bar zu verdichten.

Insbesondere ist das Sauerstoffgaserzeugungsgerät vorzugsweise dazu eingerichtet, das erzeugte Gas am Ausgang bzw. mindestens an einem der (ggf. vorhandenen) mehreren Ausgänge mit gleichbleibendem Druck abzugeben, insbesondere mit einem (gerätebestimmten) Maximaldruck.

Der Maximaldruck kann dabei vorzugsweise mindestens 5 bar, mindestens 5,5 bar, mindestens 6 bar oder sogar mindestens 6,5 bar betragen. Damit kann ein entsprechend hoher Bedarf an Sauerstoffgas gedeckt, insbesondere beispielsweise in medizinischen Notfällen eine Mehrzahl an Patienten gleichzeitig versorgt werden.

Der mindestens eine Speicher ist vorzugsweise dazu eingerichtet, erzeugtes Gas für eine Abgabe mit dem (gerätebestimmten) Maximaldruck über einen Zeitraum von mindestens 10 Minuten oder mindestens 15 Minuten vorzuhalten, also auch bei nicht nachströmendem Sauerstoffgas (beispielsweise infolge eines Stromausfalls oder eines Defekts) eine entsprechende Abgabe zu ermöglichen. So wird Anwendern in entsprechenden Situationen Zeit gegeben, geeignete Maßnahmen zu ergreifen, um die Sauerstoffversorgung zu sichern, beispielsweise mittels eines anderen Sauerstoffgaserzeugungsgeräts oder mindestens einer Sauerstoffflasche.

Gemäß vorteilhaften Ausführungsformen umfasst der Auslass/ mindestens einer der Auslässe einen Druckregler zur Einstellung eines jeweiligen Abgabedrucks des erzeugten Gases (bis zum Maximaldruck). So kann ein Verwender des Sauerstoffbereitstellungssystems den jeweiligen Druck an eine jeweils aktuelle Situation, im Falle medizinischer Anwendung beispielsweise an eine Anzahl an zu behandelnden Patienten anpassen.

Der Auslass / mindestens einer der Auslässe des Sauerstoffgaserzeugungsgeräts eines erfindungsgemäßen Sauerstoffbereitstellungssystems kann vorzugsweise über einen jeweiligen Schraub-, Schnapp- und/oder medizinischen Normanschluss an mindestens ein medizinisches Gerät wie insbesondere ein Anästhesiegerät und/oder eine Beatmungsmaske anschließbar sein, insbesondere an mehrere gleiche oder voneinander verschiedene medizinische Geräte gleichzeitig.

Alternativ oder zusätzlich kann das Sauerstoffgaserzeugungsgerät ein Füllsystem für Sauerstoffflaschen umfassen und/oder über einen jeweiligen Schraub-, Schnapp- und/oder medizinischen Normanschluss an ein solches Füllsystem anzuschließen sein. So kann das Sauerstoffbereitstellungssystem nach Wahl eines Verwenders auch zur Bevorratung mit Sauerstoff genutzt werden und/oder es ermöglichen, größere räumliche Abstände zu überbrücken.

Gemäß vorteilhaften Ausführungsformen der vorliegenden Erfindung weist das Sauerstoffgaserzeugungsgerät mindestens einen Kompressor auf, der dazu eingerichtet ist, die Umgebungsluft, ehe sie der mindestens einen Adsorbereinrichtung zugeführt wird, auf einen Druck zu komprimieren, der wenigstens 1 bar, wenigstens 1,5 bar oder wenigstens 2,4 bar über einem jeweils herrschenden Umgebungsdruck liegt. Damit kann eine besonders gute Effizienz der Adsorbereinrichtung und so eine hohe Leistung des Sauerstoffgaserzeugungsgeräts erreicht werden.

Gemäß vorteilhaften Ausführungsformen der vorliegenden Erfindung ist die mindestens eine Adsorbereinrichtung als Mehrkammer-Adsorbereinrichtung ausgebildet, die also eine Mehrzahl an jeweils mindestens ein Adsorbens (insbesondere mindestens ein Zeolith) enthaltenden Adsorberkammern aufweist. Die Adsorberkammern können dabei beispielsweise zylindrisch oder prismenförmig ausgebildet sein. Die Mehrzahl kann beispielsweise mindestens 12 oder mindestens 18 betragen.

Die Anzahl, Größe und/oder Form der Adsorberkammern und/oder das jeweils enthaltene Adsorbens ist/sind vorzugsweise so gewählt, dass bei einer Temperatur von 25 °C, einer relativen Luftfeuchte von 60 % und einem Umgebungsdruck von 1,013 bar mit dem Sauerstoffgaserzeugungsgerät aus mindestens 12 Liter eingesaugter Luft mindestens 1 Liter des Gases zu erzeugen sind.

Vorzugsweise weist/weisen die Mehrkammer-Adsorbereinrichtung/en dabei eine Schließvorrichtung auf, die dazu eingerichtet ist, nacheinander jeweilige Eingänge der mehreren Adsorberkammern zur Bedrückung/Befüllung mit komprimierter Umgebungsluft zu öffnen sowie jeweilige Ausgänge der Adsorberkammern abhängig von einer jeweiligen Adsorbens-Sättigung zu öffnen oder zu schließen. Insbesondere kann eine solche Schließvorrichtung einen Schrittmotor umfassen.

Beispielsweise kann die Schließvorrichtung bei derartigen Ausführungsformen dazu eingerichtet sein, jeweils für zwei (wechselnde) Drittel der Adsorberkammern einen jeweiligen Ausgang für das Sauerstoffgas (als leichtes Gas) zu öffnen, während gleichzeitig beim jeweiligen dritten Drittel der Adsorberkammern ein Ausgang für das Restgas (als schweres Gas bzw. Abluft) geöffnet ist.

Gemäß vorteilhaften Ausführungsformen der vorliegenden Erfindung weist das Sauerstoffgaserzeugungsgerät eine Abluftführung für bei der Druckwechsel-Adsorption entstehende Abluft auf. Die Abluftführung leitet dabei die Abluft in einen Geräteinnenraum des Sauerstoffgaserzeugungsgeräts und/oder an mindestens eine Geräteelektronik des Sauerstoffgaserzeugungsgeräts, insbesondere kann sie so ausgebildet sein, dass eine solche Geräteelektronik mindestens teilweise von der Abluft umspült wird. Auf diese Weise kann die Abluft zur Kühlung genutzt werden, so dass weitere Kühlungsvorrichtungen entbehrlich sind oder verkleinert werden können. Die Abluftführung kann insbesondere mindestens bereichsweise eine Rohrleitung umfassen und/oder mindestens bereichsweise durch mindestens einen Zwischenraum im Sauerstoffgaserzeugungsgerät gebildet sein, beispielsweise zwischen mindestens einem Gehäuseabschnitt des Sauerstoffgaserzeugungsgeräts und mindestens einer Komponente (wie insbesondere der mindestens einen Adsorbereinrichtung oder mindestens einem Kompressor des Sauerstoffgaserzeugungsgeräts).

Bei einer für die Verwendung vorgesehenen Ausrichtung des Sauerstoffbereitstellungsgeräts kann die Geräteelektronik beispielsweise über der mindestens einen Adsorbereinrichtung angeordnet sein, insbesondere in einem (vorzugsweise abklappbaren) Gehäusedeckel des Sauerstoffgaserzeugungsgeräts. So kann eine Abluftführung realisiert sein, die an einem oben liegenden Ausgang der mindestens einen Adsorbereinrichtung austretende Abluft direkt zur Geräteelektronik leitet.

Die Geräteelektronik kann beispielsweise mindestens einen Teil einer Rechnereinheit (insbesondere der weiter unten beschriebenen Rechnereinheit) bilden, beispielsweise zur Steuerung, Regelung und/oder Kontrolle der mindestens einen Adsorbereinrichtung und/oder des Verdichters und/oder einer ggf. vom Sauerstoffgaserzeugungsgerät umfassten Eingabe- und/oder Ausgabeeinrichtung.

Die Abluftführung kann mindestens einen Ventilator zum Abführen der Abluft aus dem Geräteinnenraum in eine Umgebung des Sauerstoffgaserzeugungsgeräts umfassen. Damit kann ein Abluftstrom verlängert bzw. beschleunigt werden, der eine besonders effiziente Kühlung bewirkt.

Gemäß vorteilhaften Ausführungsformen umfasst der mindestens eine Speicher eine Rohrschlange zur Aufnahme des erzeugten Gases. Die Rohrschlange kann insbesondere eine in einer Ebene verlaufende Wende und/oder mindestens eine dreidimensional verlaufende Windung (beispielsweise entlang einer Helix) umfassen. So können eine kompakte Form des Speichers und gleichwohl ein relativ hohes Volumen zur Gasaufnahme erreicht werden. Die Rohrschlange kann mindestens teilweise aus Kupfer bestehen.

Vorzugsweise ist die Rohrschlange innerhalb einer Isolierung des Speichers angeordnet. Damit kann eine Kondensatbildung vermieden oder zumindest reduziert werden, die in Bezug auf den Taupunkt auch angesichts der mit der Verdichtung einhergehenden Abkühlung der Umgebungsluft problematisch ist.

Gemäß vorteilhaften Ausführungsformen umfasst mindestens ein Einlass des Sauerstoffgaserzeugungsgeräts einen Ansaugstutzen. Damit kann ein besonders vorteilhafter Luftstrom zur mindestens einen Adsorbereinrichtung bzw. - in entsprechenden Ausführungsformen - zum mindestens einen Kompressor generiert werden. Alternativ oder zusätzlich kann ein/der mindestens eine Einlass für die Umgebungsluft mindestens einem HEPA-Filter und/oder mindestens einem ABC-Filter umfassen. Damit kann in besonders problematischen Umgebungen verhindert werden, dass das erzeugte Gas toxische Stoffe enthält.

Das Sauerstoffgaserzeugungsgerät kann vorzugsweise ein Gehäuse aufweisen, das insbesondere einen abklappbaren Gehäusedeckel (wie er oben erwähnt ist) umfassen kann. So können die verschiedenen Komponenten des Sauerstoffgaserzeugungsgeräte einerseits geschützt sein, andererseits zugänglich bleiben, beispielsweise für Reparaturfälle. Das Gehäuse kann vorzugsweise teilweise oder vollständig aus einem Leichtmetall wie Aluminium und/oder einer oder mehreren Leichtmetalllegierung/en wie Aluminiumlegierung/en bestehen.

Gemäß vorteilhaften Ausführungsformen weist das Sauerstoffgaserzeugungsgerät zur Anzeige und/oder Einstellung von mindestens einem Betriebsparameter und/oder einer Funktion des Sauerstoffgaserzeugungsgeräts mindestens ein Ein- und/oder Ausgabemittel wie insbesondere mindestens einen Drehknopf, mindestens einen Schalter und/oder mindestens ein - vorzugsweise berührungssensitives-Anzeigefeld (insbesondere einen Bildschirm) auf. Ein solches Anzeigefeld kann insbesondere derart abdunkelbar sein, dass zum Erkennen der angezeigten Information ein Nachtsichtgerät erforderlich ist. So kann beispielsweise bei Verwendung in militärischem Kontext verhindert werden, dass das Sauerstoffbereitstellungssystem und damit sein Verwender unerwünscht bemerkt wird.

Vorzugsweise umfasst das Sauerstoffgaserzeugungsgerät mindestens eine Rechnereinheit.

In den oben genannten Ausführungsformen mit Ein- und/oder Ausgabemittel kann/können diese/s insbesondere mit einer solchen Rechnereinheit verbunden sein und so als Schnittstelle zur Kommunikation mit einem Verwenders dienen. Alternativ oder zusätzlich kann die Rechnereinheit einen kabellosen und/oder kabelgebundenen Verbindungsanschluss zur einseitigen oder beidseitigen Kommunikation mit einer externen Rechnereinheit umfassen (die dann vorzugsweise über entsprechende Ein- und/oder Ausgabemittel verfügt). Insbesondere wird so jeweils einem Verwender ermöglicht, das Sauerstoffgaserzeugungsgerät zu kontrollieren bzw. dessen Betrieb an jeweilige Erfordernisse anzupassen.

Alternativ oder zusätzlich kann die Rechnereinheit beispielsweise einer Steuerung, Regelung und/oder Kontrolle mindestens einer Komponente des Sauerstoffgaserzeugungsgeräts, beispielsweise der mindestens einen Adsorbereinrichtung und/oder des Verdichters dienen.

Vorzugsweise umfasst das Sauerstoffgaserzeugungsgerät mindestens eine Kontrolleinheit zur Überwachung mindestens einer Funktion des Sauerstoffgaserzeugungsgeräts und/oder zur Messung eines Sauerstoffgehaltes und/oder CO-Messung im erzeugten Gas während dessen fortgesetzter Erzeugung (bzw. Vermehrung) und/oder während dessen Abgabe. Eine solche Kontrolleinheit kann insbesondere mindestens einen Sensor umfassen. Der/die Sensor/en kann/können mit einer Rechnereinheit (insbesondere der oben genannten Rechnereinheit) verbunden sein, die dazu eingerichtet sein kann, jeweils erfasste Werte auszuwerten und/oder auszugeben und/oder an eine externe Einheit zu übermitteln.

Gemäß vorteilhaften Ausführungsformen weist das Sauerstoffgaserzeugungsgerät eine vorgesehene Standfläche mit einer Abmessung von höchstens 1000mm^{∗} 700mm, bevorzugter von höchstens 900mm^{∗}600mm auf. Alternativ oder zusätzlich kann eine Höhe des Sauerstoffgaserzeugungsgeräts (in seiner für die Verwendung vorgesehenen Ausrichtung und einem für die Verwendung vorgesehenen Zustand, in den oben genannten Ausführungsformen mit abklappbarem Deckel insbesondere in geschlossenen Zustand) vorzugsweise maximal 900mm, bevorzugter maximal 800mm betragen. Derartige Ausführungsvarianten bilden besonders kompakte Sauerstoffgaserzeugungsgeräte, die damit auch mit einfachen Mitteln gut zu transportieren sind und die zudem auch unter beengten Voraussetzungen verwendet werden können.

In vorteilhaften Varianten umfasst ein erfindungsgemäßes Sauerstoffbereitstellungssystem eine Transportkiste, in dem das Sauerstoffgaserzeugungsgerät angeordnet ist oder angeordnet werden kann, die also insbesondere einen Aufnahmeraum für zumindest einen Teil des Sauerstoffgaserzeugungsgeräts ausbildet. Bei Anordnung des Sauerstoffgaserzeugungsgeräts im Aufnahmeraum liegt das Sauerstoffgaserzeugungsgerät vorzugsweise an einander gegenüberliegenden Seiten an einer jeweiligen Wand der Transportkiste an. So kann das Risiko eines Verrutschens des Sauerstoffgaserzeugungsgeräts innerhalb des Aufnahmeraums und damit eine Beschädigung des Sauerstoffgaserzeugungsgerät beim Transport reduziert werden.

Die Transportkiste kann insbesondere ein Unterteil und einen davon abnehmbaren und/oder abklappbaren Deckel umfassen. Im verschlossenen Zustand der Transportkiste ist der Deckel dann vorzugsweise am Unterteil fixierbar.

Insbesondere ist die Transportkiste vorzugsweise luftdicht und/oder sanddicht verschließbar und/oder spritzwassergeschützt oder sogar wasserdicht verschließbar. Ein in der Transportkiste angeordnetes Sauerstoffgaserzeugungsgerät kann so besonders gut geschützt und sein Transport damit erleichtert werden. Insbesondere kann die Transportkiste dazu eingerichtet sein, im Wasser mitsamt dem darin angeordneten Sauerstoffgaserzeugungsgerät zu schwimmen.

Gemäß vorteilhaften Ausführungsformen besteht die Transportkiste ganz oder teilweise aus Kunststoff, insbesondere Polyesterfaserstoff. Damit können eine besonders gute Beständigkeit und zugleich eine relativ geringe Masse der Transportkiste erreicht werden. Vorzugsweise weist die (leere) Transportkiste eine Masse von höchstens 35kg, bevorzugter höchstens 30kg auf.

Die Transportkiste kann insbesondere gemäß Militärstandard ausgebildet, beispielsweise nach 15MIL-Spec- und/oder MIL-STD 810 zertifiziert sein. Vorzugsweise ist sie mindestens teilweise mit einem elastischen Material zur Stoßdämpfung ausgekleidet; auf diese Weise kann ein besonders guter Schutz des Sauerstoffgaserzeugungssystem bei seinem Transport gewährleistet werden. Alternativ oder zusätzlich kann die Transportkiste innen mindestens teilweise mit einem Material zur Schallisolierung bzw. -dämmung versehen sein. So kann eine Geräuschemission des Sauerstoffgaserzeugungsgeräts besonders gering gehalten werden, wenn es in der (ggf. geöffneten) Transportkiste (bzw. in deren Unterteil) betrieben wird.

Gemäß vorteilhaften Ausführungsformen weist die Transportkiste eine quaderartige Grundform auf. Damit ist sie gut stapel- und verstaubar und kann insbesondere zusammen mit anderen Transportkisten raumsparend angeordnet werden, beispielsweise zum Transport.

Vorzugsweise weist die Transportkiste mindestens zwei Griffe auf. In entsprechenden Ausführungsformen können die Griffe jeweils am Deckel und/oder am Unterteil angeordnet sein. Vorteilhaft sind insbesondere Ausführungsformen, bei denen die Griffe eine bewegliche Aufhängung haben, insbesondere (relativ zu Deckel und/oder Unterteil) verschwenkbar sind. So können sie bei ihrer Verwendung zum Heben oder Tragen der Transportkiste (ggf. mit darin angeordnetem Sauerstoffgaserzeugungsgerät) zur verbesserten Greifbarkeit von einer Wand der Transportkiste abgeklappt und andererseits an die Wand angelegt werden, wenn das Sauerstoffbereitstellungssystem besonders kompakt verstaut werden soll.

Gemäß vorteilhaften Ausführungsformen weist ein erfindungsgemäßes Sauerstoffbereitstellungssystem mindestens eine Rolle auf, mittels deren das Sauerstoffgaserzeugungsgerät verfahren und damit umpositioniert werden kann. Die mindestens eine Rolle kann dabei dazu eingerichtet sein, das Verfahren (Umpositionieren) in einem unangehobenen Zustand des Sauerstoffgaserzeugungsgeräts zu ermöglichen und/oder in einem teilweise, beispielsweise einseitig angehobenen Zustand.

Mindestens eine solche Rolle kann am Sauerstoffgaserzeugungsgerät, insbesondere an einem Gehäuse desselben angeordnet sein. Damit kann eine vereinfachte Umpositionierung des Sauerstoffgaserzeugungsgeräts erreicht werden. Weist das Sauerstoffbereitstellungssystem eine Transportkiste wie oben beschrieben auf, kann (alternativ oder zusätzlich) mindestens eine Rolle an der Transportkiste angeordnet sein. Damit kann ein Umpositionieren der Transportkiste mit darin angeordnetem Sauerstoffgaserzeugungsgerät erleichtert werden.

Die mindestens eine Rolle kann eine einstellbare, insbesondere ausklappbare Aufhängung aufweisen, beispielsweise eine relativ zum Sauerstoffgaserzeugungsgerät bzw. zur Transportkiste verschwenkbare Achse. So kann das Sauerstoffbereitstellungssystem zum Verstauen eine besonders kompakte Form gebracht werden.

Ein erfindungsgemäßes Verfahren dient dem Bereitstellen eines Sauerstoffgases, das zu mindestens 90 Vol.-% aus Sauerstoff besteht. Das Verfahren umfasst dabei ein Betreiben eines Sauerstoffbereitstellungssystems gemäß einer Ausführungsform der vorliegenden Erfindung.

Insbesondere kann das Verfahren ein Beatmen einer Mehrzahl an Patienten mittels einer jeweiligen Atemmaske umfassen, die an das Sauerstoffgaserzeugungsgerät des Sauerstoffbereitstellungssystems angeschlossen sind. Die Mehrzahl kann beispielsweise fünf oder mehr Patienten umfassen.

Im Folgenden werden bevorzugte Ausführungsbeispiele der Erfindung anhand von Zeichnungen näher erläutert. Es versteht sich, dass einzelne Elemente und Komponenten auch anders kombiniert werden können als dargestellt.

Es zeigen:
- Fig. 1:: eine schematische Darstellung eines Sauerstoffgaserzeugungsgeräts einer ersten exemplarischen Ausführungsform eines erfindungsgemäßen Sauerstoffbereitstellungssystems;
- Fig. 2:: eine schematische Darstellung eines Sauerstoffgaserzeugungsgeräts einer zweiten exemplarischen Ausführungsform eines erfindungsgemäßen Sauerstoffbereitstellungssystems; und
- Fig. 3:: eine Transportkiste einer exemplarischen Ausführungsform eines erfindungsgemäßen Sauerstoffbereitstellungssystems.

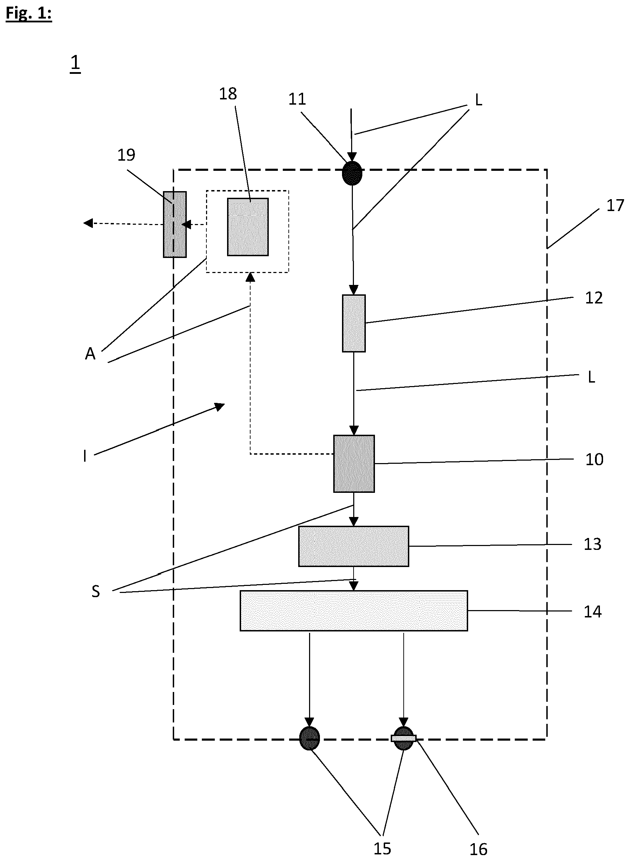

Die Figur 1 zeigt ein Aufbauschema eines ersten Ausführungsbeispiels eines mobilen Sauerstoffgaserzeugungsgeräts 1 eines erfindungsgemäßen Sauerstoffbereitstellungssystems und illustriert damit den Ablauf einer exemplarischen Ausführungsvariante eines erfindungsgemäßen Verfahrens. Vorzugsweise hat das Sauerstoffgaserzeugungsgerät 1 eine Masse von höchstens 90kg oder höchstens

Das Sauerstoffgaserzeugungsgerät 1 umfasst dabei ein Gehäuse 17 mit einem Einlass 11 für Umgebungsluft L. Vom Einlass 11 wird die eingetretene Umgebungsluft L zu einem Kompressor 12 geleitet, der sie vorzugsweise auf einen Druck komprimiert, der wenigstens 1 bar, wenigstens 1,5 bar oder wenigstens 2,4 bar über einem jeweils herrschenden Umgebungsdruck liegt.

Die derart komprimierte Luft wird dann einer (vorliegend einzelnen) Adsorbereinrichtung 10 zugeführt, die aus ihr mittels Druckwechsel-Adsorption ein Gas S erzeugt, das zu mindestens 90 Vol.- % aus Sauerstoff besteht. Vorzugsweise ist die Adsorbereinrichtung 10 dabei als Mehrkammer-Adsorbereinrichtung mit einer Mehrzahl an jeweils mindestens ein Adsorbens (insbesondere mindestens ein Zeolith) enthaltenden Adsorberkammern ausgebildet; dabei kann sie insbesondere eine Schließvorrichtung aufweisen, wie oben beschrieben.

Das erzeugte (Sauerstoff-)Gas S wird in einem Verdichter 13 verdichtet, und zwar vorzugsweise auf einen Druck von mindestens 5 bar, mindestens 5,5 bar, mindestens 6 bar oder mindestens 6,5 bar. Darauf wird das verdichtete erzeugte Gas in (vorliegend einem einzelnen) Speicher gespeichert, der vorzugsweise mindestens eine Rohrschlange zur Aufnahme des erzeugten Gases S umfasst und von dem aus es über (vorliegend zwei) Auslässe 15 abgegeben werden kann. Einer der Auslässe 15 weist dabei vorliegend einen Druckregler 16 auf, der dazu eingerichtet ist, einen jeweils gewünschten Abgabedruck für das erzeugte Sauerstoffgas S einzustellen.

Insbesondere kann mindestens einer der Auslässe 15 vorzugsweise an ein Füllsystem für Sauerstoffflaschen und/oder an ein medizinisches Gerät (wie insbesondere ein Anästhesiegerät und/oder eine Beatmungsmaske) anschließbar sein (nicht dargestellt). Eine Regelung durch den Druckregler 16 kann dann (automatisch oder durch einen Verwender eingestellt) in Abhängigkeit von einer Anzahl und/oder einer Art angeschlossener Geräte erfolgen.

Das Gehäuse des in der Figur 1 schematisch dargestellten Sauerstoffgaserzeugungsgeräts 1 umgibt den Kompressor 12, die Adsorbereinrichtung 10, den Verdichter 13 und den Speicher 14 sowie eine im Geräteinnenraum I angeordnete Geräteelektronik 18 des Sauerstoffgaserzeugungsgeräts 1.

Eine solche Geräteelektronik 18 kann (ggf. zusammen mit mindestens einer weiteren elektronischen Komponente) insbesondere mindestens einen Teil einer Rechnereinheit bilden, die beispielsweise einer Steuerung, Regelung und/oder Kontrolle der Adsorbereinrichtung 10, des Kompressors 12, des Verdichters 13, des Speichers 14 und/oder des Druckreglers 16 dienen kann und/oder einer Kommunikation mit einem Verwender über eine (nicht dargestellte) Eingabe- und/oder Ausgabeeinheit.

Wie dem Schema der Figur 1 zu entnehmen ist, wird bei der Druckwechsel-Adsorption in der Adsorbereinrichtung 11 entstehende Abluft A in den Geräteinnenraum I geführt, insbesondere zur Geräteelektronik 18, die vorliegend von der Abluft A umströmt und dadurch gekühlt wird. Die Abluft A wird dann, gefördert durch mindestens eines Ventilator 19, durch einen Ausgang des Gehäuses 17 in eine Umgebung des Sauerstoffgaserzeugungsgerät 1 abgelassen.

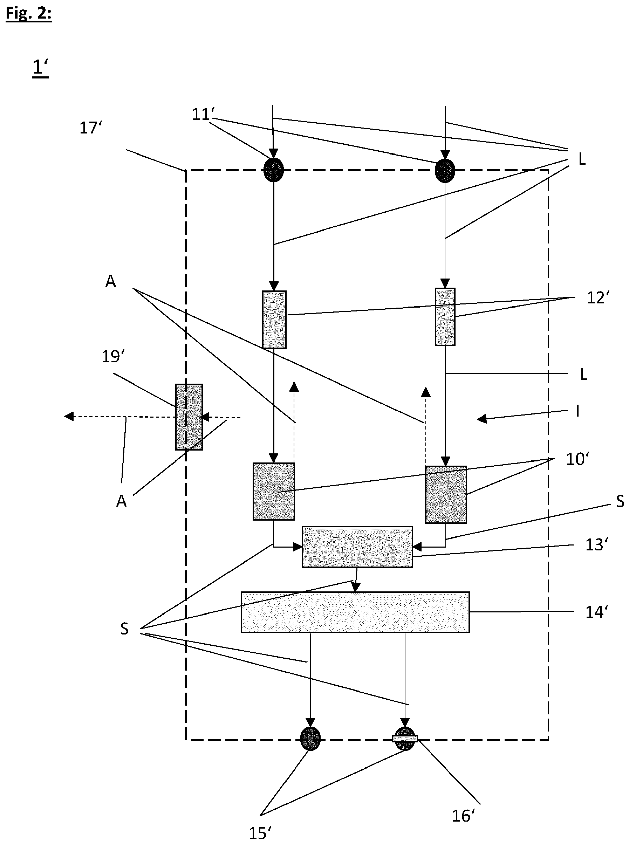

Die Figur 2 zeigt schematisch ein Aufbauschema eines zweiten Ausführungsbeispiels eines Sauerstoffgaserzeugungsgeräts 1' eines erfindungsgemäßen Sauerstoffbereitstellungssystems. Das Sauerstoffgaserzeugungsgerät 1' weist dabei zwei Adsorbereinrichtungen 10' auf, die vorzugsweise dazu eingerichtet sind, einzeln (nacheinander) oder synchron zu arbeiten. Vorzugsweise ist mindestens eine der Adsorbereinrichtungen 10' dabei als Mehrkammer-Adsorbereinrichtung mit einer Mehrzahl an jeweils mindestens ein Adsorbens (insbesondere mindestens ein Zeolith) enthaltenden Adsorberkammern ausgebildet; dabei kann sie insbesondere eine Schließvorrichtung aufweisen, wie oben beschrieben.

Die Adsorbereinrichtungen 10' werden dabei jeweils mit Umgebungsluft beschickt, die durch einen jeweiligen Einlass 11' hindurch in einen jeweiligen Kompressor 12' eingetreten ist und die von diesem dann vorzugsweise auf einen Druck komprimiert wurde, der wenigstens 1 bar, wenigstens 1,5 bar oder wenigstens 2,4 bar über einem jeweils herrschenden Umgebungsdruck liegt.

Das von den Adsorbereinrichtungen 12' erzeugte (Sauerstoff-)Gas S wird vorliegend einem gemeinsamen Verdichter 14' zugeführt, der es entsprechend dem Obigen auf vorzugsweise einen Druck von mindestens 5 bar, mindestens 5,5 bar, mindestens 6 bar oder mindestens 6,5 bar verdichtet, ehe es im Speicher 14' gespeichert wird. Über (vorliegend zwei) Auslässe 15', von denen vorliegend einer einen Druckregler 16' zur Einrichtung eines gewünschten Abgabedrucks für das erzeugte Sauerstoffgas S aufweist, kann das erzeugte Gas entsprechend dem Obigen abgegeben werden.

Ein Gehäuse 17' umgibt die Kompressoren 12', die Adsorbereinrichtungen 10', den Verdichter 13' und den Speicher 14'

Von den Adsorbereinrichtungen 10' jeweils abgegebene Abluft A wird in den Innenraum I' des Sauerstoffgaserzeugungsgeräts 1' geführt und mittels mindestens einem Ventilator 19' durch einen Auslass im Gehäuse 17' abgegeben. Vorzugsweise führt ein dabei entstehender Abluftstrom der Abluft A an einer Geräteelektronik vorbei und kühlt sie so; aus Übersichtsgründen ist die Geräteelektronik in der Figur 2 nicht dargestellt.

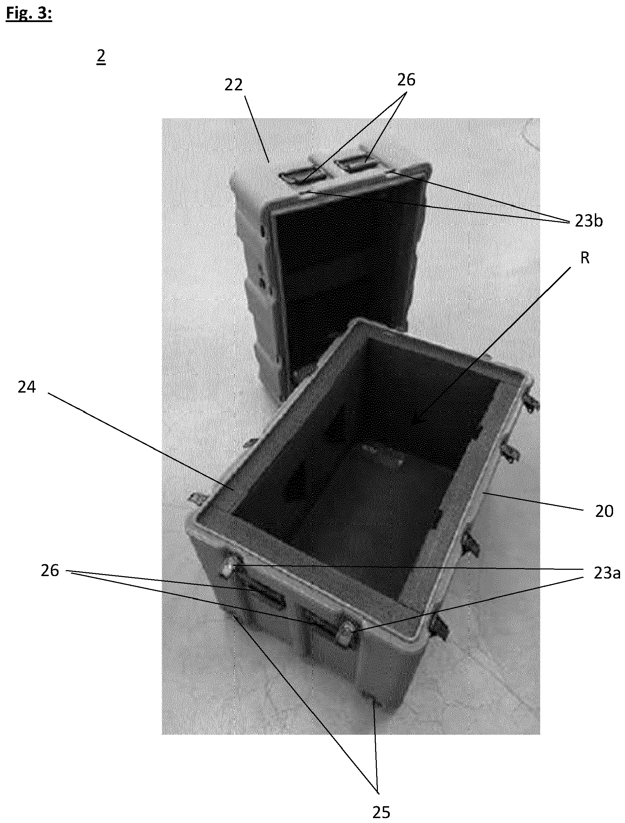

Die Figur 3 zeigt eine Transportkiste 2 eines erfindungsgemäßen Sauerstoffbereitstellungssystems gemäß einem Ausführungsbeispiel. Die Transportkiste 2 weist eine quaderartige Grundform auf und umfasst dabei ein Unterteil 20 sowie einen von diesem vorliegend vollständig abnehmbaren Deckel 21. Mittels Klemmen 22a und jeweils zugehöriger Klemmeingriffe 22b (von denen in der Figur 3 aus Übersichtsgründen nur je zwei mit Bezugszeichen versehen sind) kann der Deckel 21, wenn er auf das Unterteil 20 aufgesetzt ist, an diesem fixiert werden. Vorzugsweise ist die so geschlossene Transportkiste 2 luftdicht und/oder sanddicht und/oder spritzwassergeschützt (oder sogar wasserdicht).

Die Transportkiste 2 bildet einen Aufnahmeraum R für ein in der Figur 3 nicht gezeigtes Sauerstoffgaserzeugungsgerät des Sauerstoffbereitstellungssystems;
bei Anordnung des Sauerstoffgaserzeugungsgeräts im Aufnahmeraum R liegt das Sauerstoffgaserzeugungsgerät vorzugsweise an mindestens zwei einander gegenüberliegenden Seiten an einer jeweiligen Wand der Transportkiste 2 an, die vorliegend mit einem elastischen Material 23 zur Stoßdämpfung ausgekleidet ist; vorzugsweise dient das elastische Material 23 dabei zugleich einer Schallisolierung bzw. -dämmung.

Die Transportkiste 2 weist weiterhin vorliegend vier Rollen 24 auf, von denen in der Figur 3 aufgrund der Perspektive nur zwei sichtbar sind. Vorzugsweise weisen die Rollen eine verstellbare Aufhängung auf, so dass sie zum Verstauen der Transportkiste bzw. des Sauerstoffbereitstellungssystems in einer Transportkistenwand versenkt und zu ihrer Verwendung ganz oder teilweise aus ihrer Versenkung bewegt werden können. Das Versenken kann ein unerwünschtes Verrollen verhindern, zudem ermöglicht es ein besonders raumsparendes Verstauen des Sauerstoffbereitstellungssystems.

Die Transportkiste kann auf allen vier Rollen verfahrbar sein und/oder (in einem einseitig angehobenen Zustand) auf nur zwei der Rollen.

Sowohl am Unterteil 20 als auch am Deckel 21 sind bei der dargestellten Ausführungsform Griffe verschwenkbar angeordnet, an denen vorzugsweise die Transportkiste mit einem darin anzuordnenden Sauerstoffgaserzeugungsgerät angehoben und getragen und/oder beim Rollen gezogen werden kann. Im ihrem der Figur 3 gezeugten eingeschwenkten Zustand sind die Griffe dabei in eine jeweilige Außenwand des Unterteils 20 bzw. Deckels 21 eingelassen, so dass sie nicht überstehen und damit bei einem Verstauen der Transportkiste nicht hinderlich sind.

Offenbart ist ein Sauerstoffbereitstellungssystem mit einem mobilen Sauerstoffgaserzeugungsgerät 1. Das Sauerstoffgaserzeugungsgerät 1 umfasst mindestens eine Adsorbereinrichtung 10, die dazu eingerichtet ist, mittels Druckwechsel-Adsorption aus Umgebungsluft L ein Gas S zu erzeugen, das zu mindestens 90 Vol.-% aus Sauerstoff besteht. Darüber hinaus umfasst das Sauerstoffgaserzeugungsgerät 1 mindestens einen Verdichter 13 zum Verdichten des erzeugten Gases S, mindestens einen Speicher 14 zur Aufnahme des vom mindestens einen Verdichter verdichteten Gases und einen oder mehrere Auslässe 15 zur Abgabe des erzeugten Gases aus dem mindestens einen Speicher 14.

Offenbart ist ferner ein Verfahren Bereitstellen eines Sauerstoffgases S, wobei das Verfahren ein Betreiben eines Sauerstoffbereitstellungssystems umfasst.

### Bezugszeichen

- 1, 1': Sauerstoffgaserzeugungsgerät
- 10, 10': Adsorbereinrichtung
- 11, 11': Einlass
- 12, 12': Kompressor
- 13, 13': Verdichter
- 14, 14': Speicher
- 15, 15': Auslass
- 16, 16': Druckregler
- 17, 17': Gehäuse
- 18: Geräteelektronik
- 19, 19': Ventilator

- 2: Transportkiste
- 20: Unterteil
- 21: Deckel
- 22a: Klemme
- 22b: Klemmeingriff
- 23: elastisches Material
- 24: Rolle

- A: Abluft
- I: Innenraum
- L: Umgebungsluft
- R: Aufnahmeraum
- S: erzeugtes Gas

## Patentansprüche

1. Sauerstoffbereitstellungssystem mit einem mobilen Sauerstoffgaserzeugungsgerät (1, 1'), das umfasst:
- mindestens eine Adsorbereinrichtung (10, 10'), die dazu eingerichtet ist, mittels Druckwechsel-Adsorption aus Umgebungsluft (L) ein Gas (S) zu erzeugen, das zu mindestens 90 Vol.-% aus Sauerstoff besteht;
- mindestens einen Verdichter (13, 13') zum Verdichten des erzeugten Gases (S),
- mindestens einen Speicher (14, 14') zur Aufnahme des vom mindestens einen Verdichter verdichteten Gases und
- einen oder mehrere Auslässe (15, 15') zur Abgabe des erzeugten Gases aus dem mindestens einen Speicher (14, 14').

2. Sauerstoffbereitstellungssystem gemäß Anspruch 1, wobei der mindestens eine Speicher (14, 14') mindestens eine Rohrschlange zur Aufnahme des erzeugten Gases (S) umfasst.

3. Sauerstoffbereitstellungssystem gemäß einem der Ansprüche 1 oder 2, wobei das Sauerstoffgaserzeugungsgerät (1, 1') mindestens einen Kompressor (12, 12') umfasst, der dazu eingerichtet ist, die Umgebungsluft (L) vor ihrem Eintritt in die mindestens eine Adsorbereinrichtung (10, 10') auf einen Druck zu komprimieren, der wenigstens 1 bar, wenigstens 1,5 bar oder wenigstens 2,4 bar über einem jeweils herrschenden Umgebungsdruck liegt.

4. Sauerstoffbereitstellungssystem gemäß einem der Ansprüche 1 bis 3, wobei das Sauerstoffgaserzeugungsgerät (1, 1') mindestens einen oder mindestens zwei Mehrkammer-Adsorbereinrichtungen aufweist, der/die jeweils umfassen:
- eine Mehrzahl an Adsorberkammern, die jeweils ein Adsorbens enthalten; und
- eine Schließvorrichtung, die dazu eingerichtet ist, nacheinander jeweilige Eingänge der mehreren Adsorberkammern der jeweiligen Adsorbereinrichtung zur Bedrückung/Befüllung mit komprimierter Umgebungsluft zu öffnen sowie jeweilige Ausgänge der Adsorberkammern abhängig von einer jeweiligen Adsorbens-Sättigung zu öffnen oder zu schließen.

5. Sauerstoffbereitstellungssystem gemäß Anspruch 4, wobei die Anzahl, Größe und/oder Form der Adsorberkammern und/oder das jeweils enthaltene Adsorbens so gewählt ist/sind, dass bei einer Temperatur von 25 °C, einer relativen Luftfeuchte von 60 % und einem Umgebungsdruck von 1,013 bar mit dem Sauerstoffgaserzeugungsgerät aus mindestens 12 Liter eingesaugter Luft mindestens 1 Liter des Gases zu erzeugen sind.

6. Sauerstoffbereitstellungssystem gemäß einem der Ansprüche 4 oder 5, wobei das Sauerstoffgaserzeugungsgerät eine Abluftführung für bei der Druckwechsel-Adsorption entstehende Abluft (A) derart aufweist, dass die Abluft zur Kühlung in einen Geräteinnenraum des Sauerstoffgaserzeugungsgeräts und/oder an mindestens eine Geräteelektronik (18) geleitet wird.

7. Sauerstoffbereitstellungssystem gemäß einem der vorhergehenden Ansprüche, wobei der Verdichter dazu eingerichtet ist, das erzeugte Gases auf einen Druck von mindestens 5 bar, mindestens 5,5 bar, mindestens 6 bar oder mindestens 6,5 bar zu verdichten.

8. Sauerstoffbereitstellungssystem gemäß einem der vorhergehenden Ansprüche, wobei der Speicher dazu eingerichtet ist, im Falle eines Ausfalls der Druckwechsel-Adsorption eine Bereitstellung des erzeugten Sauerstoffgases mit mindestens 5bar über mindestens 10 Minuten oder mindestens 15 Minuten zu gewährleisten.

9. Sauerstoffbereitstellungssystem gemäß einem der vorhergehenden Ansprüche, wobei am Auslass bzw. an mindestens einem der mehreren Auslässe des Sauerstoffgaserzeugungsgeräts
- ein Ausgangsdruck des abzugebenden erzeugten Gases einstellbar ist und/oder
- über einen jeweiligen Schraub-, Schnapp- und/oder medizinischen Normanschluss mindestens ein medizinisches Gerät anschließbar ist, insbesondere an mehrere gleiche oder voneinander verschiedene medizinische Geräte gleichzeitig.

10. Sauerstoffbereitstellungssystem gemäß einem der vorhergehenden Ansprüche, wobei das Sauerstoffgaserzeugungsgerät ein Füllsystem für Sauerstoffflaschen umfasst und/oder über einen jeweiligen Schraub-, Schnapp- und/oder medizinischen Normanschluss an ein solches Füllsystem anzuschließen ist.

11. Sauerstoffbereitstellungssystem gemäß einem der vorhergehenden Ansprüche, wobei das Sauerstoffgaserzeugungsgerät eine Rechnereinheit umfasst,
- die mit mindestens Eingabemittel zur Einstellung und/oder mit einem Ausgabemittel zur Anzeige jeweils von mindestens einem Betriebsparameter und/oder einer Funktion des Sauerstoffgaserzeugungsgeräts verbunden ist und/oder
- die einen kabellosen und/oder kabelgebundenen Verbindungsanschluss zur einseitigen oder beidseitigen Kommunikation mit einer externen Rechnereinheit aufweist.

12. Sauerstoffbereitstellungssystem gemäß einem der vorhergehenden Ansprüche, wobei das Sauerstoffgaserzeugungsgerät eine Kontrolleinheit zur Überwachung mindestens einer Funktion des Sauerstoffgaserzeugungsgeräts und/oder zur Messung eines Sauerstoffgehaltes und/oder CO-Messung im erzeugten Gas während dessen fortgesetzter Erzeugung (bzw. Vermehrung) und/oder während dessen Abgabe umfasst.

13. Sauerstoffbereitstellungssystem gemäß einem der vorhergehenden Ansprüche, das zudem eine Transportkiste (2) umfasst, in dem das Sauerstoffgaserzeugungsgerät (1, 1') angeordnet oder anzuordnen ist, wobei die Transportkiste
- luftdicht und/oder wasserdicht und/oder sanddicht verschließbar ist; und/oder
- ganz oder teilweise aus Kunststoff, insbesondere Polyesterfaserstoff besteht; und/oder
- eine Masse von höchstens 35kg, bevorzugter höchstens 30kg aufweist; und/oder
- gemäß Militärstandard ausgebildet, insbesondere 15MIL-Spec- und/oder MIL-STD 810-zertifiziert ist; und/oder
- mindestens teilweise mit einem elastischen Material (24) zur Stoßdämpfung ausgekleidet ist; und/oder
- eine innere Schallisolierung aufweist, und/oder
- eine im Wesentlichen quaderförmige Form aufweist; und/oder
- einen abnehmbaren oder abklappbaren, im verschlossenen Zustand der Transportkiste fixierbaren Deckel (22) hat; und/oder
- mindestens zwei Griffe (26) aufweist.

14. Sauerstoffbereitstellungssystem gemäß einem der vorhergehenden Ansprüche, das eine, zwei oder mehr Rolle/n zum Verfahren des Sauerstoffgaserzeugungsgeräts aufweist.

15. Verfahren zum Bereitstellen eines Sauerstoffgases (S), das zu mindestens 90 Vol.-% aus Sauerstoff besteht, wobei das Verfahren ein Betreiben eines Sauerstoffbereitstellungssystems gemäß einem der vorhergehenden Ansprüche umfasst.
